# EUROPEAN PATENT SPECIFICATION

(11) **EP 2 196 150 B1**
(45) Date of publication and mention of the grant of the patent: **17.08.2016**
(21) Application number: 09177849.8
(22) Date of filing: 03.12.2009
(51) Int. Cl.: A61B 8/00

(54) **Hand-held ultrasound system**
Tragbares Ultraschallsystem
Système à ultrasons portable

(30) Priority: 08.12.2008 KR 20080124197
(43) Date of publication of application: 16.06.2010
(73) Proprietor: Samsung Medison Co., Ltd., Hongcheon-gun, Gangwon-do, 25108 (KR)
(72) Inventor: Hyun, Dong Gyu, 135-851, Seoul (KR)
(74) Representative: Schmid, Wolfgang

(56) References cited:
- US-A1- 2006 116 578
- US-A1- 2008 119 731
- US-A1- 2008 122 796
- US-B1- 6 468 212
- US-B1- 6 891 920

## Description

### TECHNICAL FIELD

The present disclosure generally relates to ultrasound systems, and more particularly to a hand-held ultrasound system.

### BACKGROUND

An ultrasound system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., human organs).

The ultrasound system, which is typically used in clinics, is relatively large and heavy. Thus, it is generally operated while being fixed at a specific location. Also, although the ultrasound system is typically manufactured as a small-sized system, its weight may exceed about 10 Kg. Thus, it may not be easy to move the ultrasound system. As such, it may be difficult to carry the ultrasound system as a portable system. Accordingly, the ultrasound systems have been developed into two types of systems, namely, a cart-based ultrasound system and a portable ultrasound system (similar to a laptop computer).

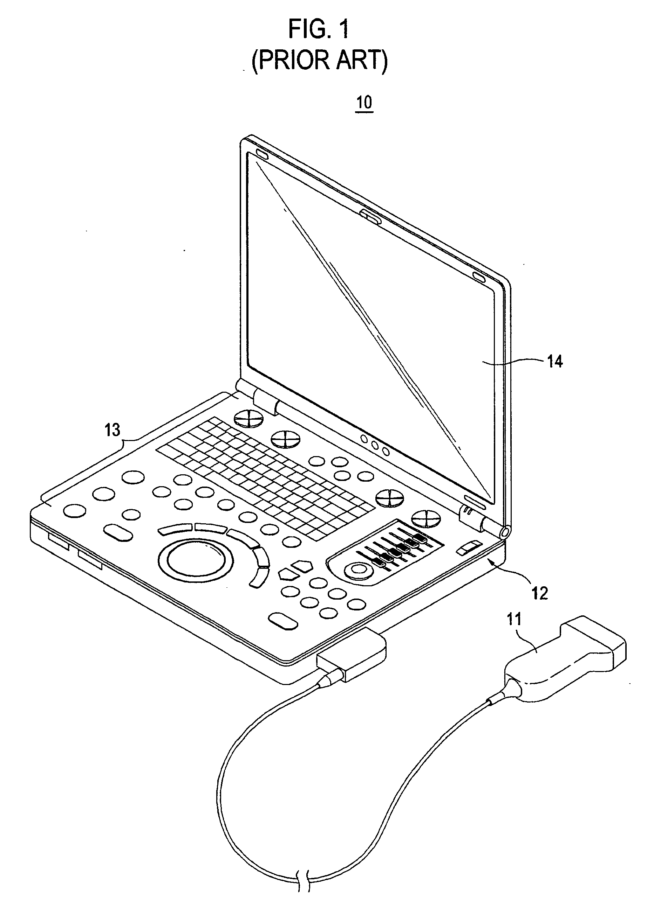

FIG. 1 is a schematic diagram showing a laptop computer-type portable ultrasound system. As shown in FIG 1, the potable ultrasound system 10 may include an ultrasound probe 11, a body 12, a control panel 13 and a display 14. The ultrasound probe 11 may transmit/receive ultrasound signals to/from a target object to output receive signals. The body 12 may include processing elements for forming ultrasound images based on the receive signals. The control panel 13 may be mounted on the body 12. The control panel 13 may include a plurality of selection buttons, knob buttons, sliders for adjusting image parameters, keyboard, track ball and the like. The display may be a liquid crystal display, organic light-emitting diode display or the like.

While the laptop computer-type portable ultrasound system may be small, there is still a strong need in the art to provide a hand-held ultrasound system, which has acceptable performance characteristics and is easy to use.

A hand-held ultrasound system according to the preamble of claim 1 is known from US 2006/116578 A1.

US 6,468,212 B1 refers to a user control interface for an ultrasound processor having a display in which an ultrasound image is displayed in a central area and a menu item is displayed in a side area.

From US 2008/119731 A1 there is known a portable ultrasound having a touch screen interface on which an operation menu is shown next to an ultrasound image.

### SUMMARY

An embodiment for providing a hand-held ultrasound system is disclosed herein. In one embodiment a hand-held ultrasound system comprises the features of claim 1.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG 1 is a schematic diagram showing a laptop computer-type portable ultrasound system.
FIG. 2 is a schematic diagram showing an illustrative embodiment of a hand-held ultrasound system.
FIG 3 is a block diagram showing an illustrative embodiment of the body.
FIG 4 is a schematic diagram showing an example of rotating a hand-held ultrasound system.
FIG. 5 is a schematic diagram showing an example of partitioning a display region of a touch screen into a plurality of display regions.
FIGS. 6A to 6C are schematic diagrams showing examples of inputting user instructions on a touch screen.
FIGS. 7 and 8 are schematic diagrams showing examples of displaying an ultrasound image according to status of a hand-held ultrasound held by a user.
FIGS. 9 to 11 are schematic diagrams showing examples of displaying a touch screen menu together with an ultrasound image.
FIG 12 is a schematic diagram showing an example of setting a region of interest on an ultrasound image.
FIG 13 is a schematic diagram showing an example of inputting a user instruction for selecting an ultrasound image and ultrasound data.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

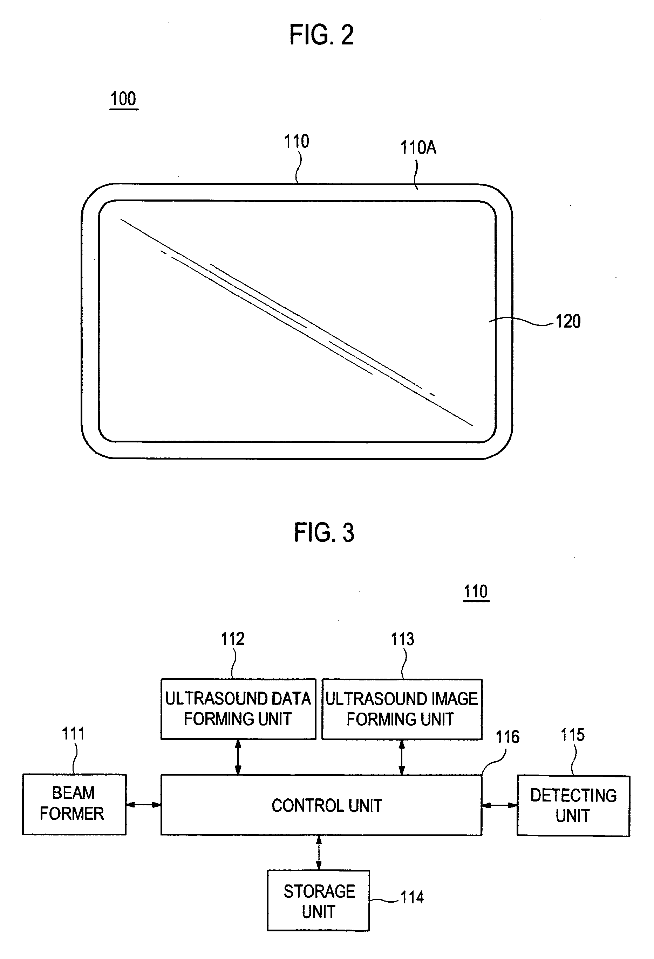

FIG. 2 is a schematic diagram showing an illustrative embodiment of a hand-held ultrasound system. The hand-held ultrasound system 100 may include a body 110 and a touch screen 120. The hand-held ultrasound system 100 may further include an ultrasound probe (not shown) that may be operable to transmit/receive ultrasound signals to/from a target object to thereby output electrical receive signals. The ultrasound probe may include an array transducer consisting of a plurality of elements. The ultrasound probe may be connected to the body 110 through wired or wireless communication.

The body 110 may be configured with housing and a plurality of processing elements packaged in the housing. The housing may be formed in a rectangular shape, although it is not limited thereto. For example, the housing may be formed to have a typical rectangular shape with its four corners rounded, as illustrated in FIG 2. In one embodiment, the body 110 may be formed to have size, weight and shape suitable for allowing a user to easily hold by using a single hand. The hand-held ultrasound system may be manufactured to have a weight of less than 1 kg. The processing elements packaged in the body 110 may receive the receive signals from the ultrasound probe to form an ultrasound image.

FIG 3 is a block diagram showing an illustrative embodiment of the processing elements packaged in the body 110. The body 110 may include a beam former 111 that may be operable to apply delays to the receive signals in consideration of distances between the elements and focal points, thereby outputting receive-focused signals. The body 110 may further include an ultrasound data forming unit 112 that may be operable to form ultrasound data based on the receive-focused signals. In one embodiment, the ultrasound data forming unit 112 may be embodied by a digital signal processor.

The body 110 may further include an ultrasound image forming unit 113 that may be operable to form ultrasound image based on the ultrasound data. In one embodiment, the ultrasound images may include at least one of a brightness mode image, a Doppler mode image, a color mode image, an elastic mode image, a 3-dimensional mode image and the like.

The body 110 may further include a storage unit 114 for storing the ultrasound data and the ultrasound images. In one embodiment, the storage unit 114 may include a first storage section (not shown) for storing the ultrasound data and a second storage section (not shown) for storing the ultrasound images. The storage unit 114 may further store preset image sets for graphic user interface. The storage unit 114 may include at least one of a hard disk, flash memory, CD ROM, DVD and the like.

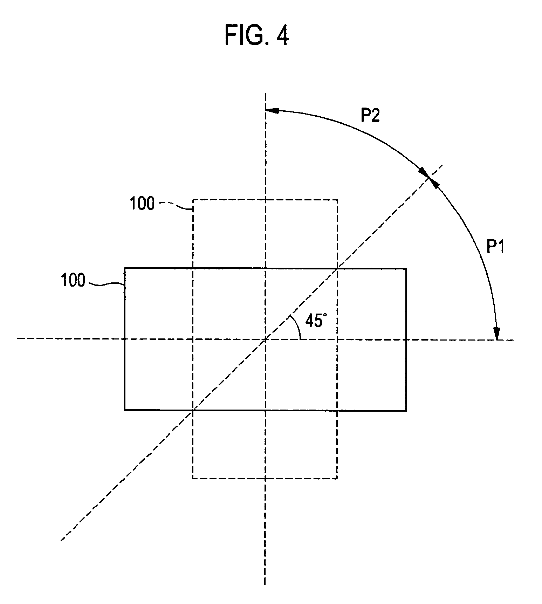

The body 110 may further include a detecting unit 115 that may be operable to detect status of the hand-held ultrasound system 100 held by a hand to thereby output a detection signal. In one embodiment, the detecting unit 115 may include sensors mounted on respective edges 110A of the body for detecting which edge is held by a hand of the user. The detecting unit 115 may be operable to output a detection signal corresponding to the touched edge. In one embodiment, the detecting unit 115 may include a gravity sensor for detecting rotation of the hand-held ultrasound system 100. In such a case, if the hand-held ultrasound system 100 is rotated within a first angle range P1 from 0 to 45 degrees with respect to a reference angle (e.g., 0 degree) as illustrated in FIG 4, then the detecting unit 115 may output a first detection signal. Also, if the hand-held ultrasound system 100 is rotated to be within a second angle range P2 from 45 to 90 degrees as illustrated in FIG 4, then the detecting unit 115 may output a second detection signal. In one embodiment, when the hand-held ultrasound system 100 is rotated to be positioned at an angle of 45 degrees, the detecting unit 115 may output the second detection signal.

The body 110 may further include a control unit 116 that may be operable to control the transmission and reception of the ultrasound signals. The control unit 116 may be further operable to control the display of the touch screen according to user instructions.

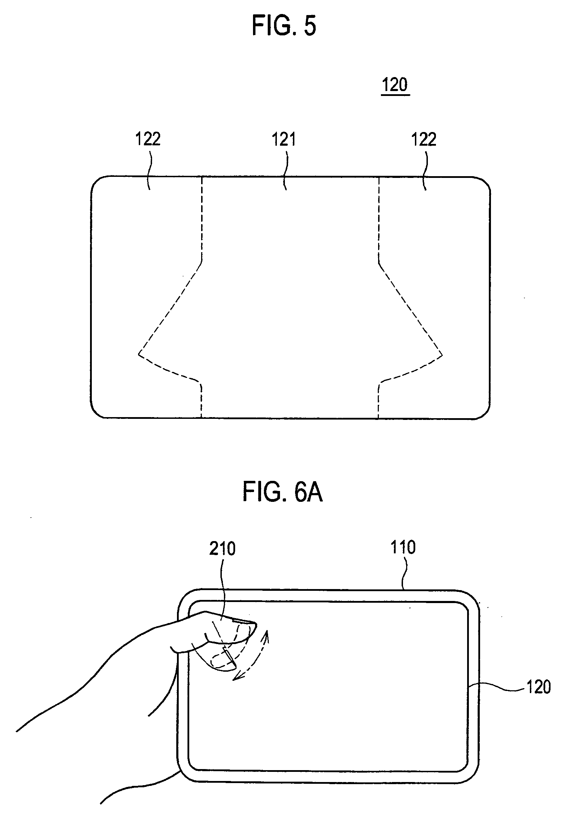

In one embodiment, the touch screen 120 may be installed on one plane of the body 110. The touch screen 120 may include a display and a touch panel with a touch responsive surface. The display may include one of liquid crystal display, organic light-emitting diode display and the like. The touch panel may be placed over a screen of the display. The touch screen 120 may be operable to display the ultrasound images. The touch screen 120 may be further operable to display a preset touch screen menu for graphic user interface by using the preset image sets stored in the storage unit 114. While watching the preset touch screen menu, the user may input user instructions onto the touch screen 120 by a finger, stylus or the like. In one embodiment, a display region of the touch screen 120 may be partitioned into first and second display regions. The first display region may be utilized to display an ultrasound image and receive user instructions such as an instruction for setting a region of interest (ROI) on an ultrasound image, an instruction for adjusting the ROI, an instruction for moving the ROI and the like. The second display region may be utilized to display the preset touch screen menu for graphic user interface. As illustrated in FIG 5, for example, the touch screen 120 may include a first display region 121 disposed at a center portion of the display region and the second display regions 122 disposed at both sides of the first display region 121.

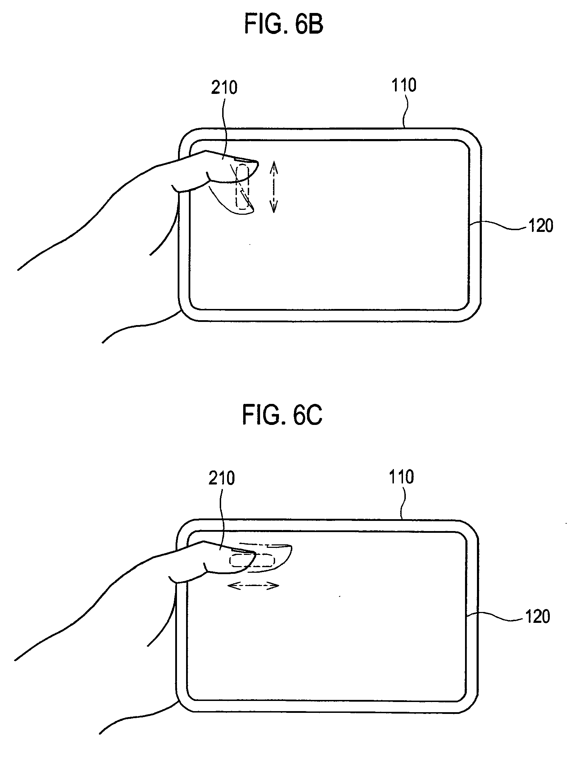

In one embodiment, the user instructions may be inputted through various events. For example, the user instructions may be inputted through a tap event (click event). Also, the user instructions may be inputted through a circular drag event as shown in FIG 6A, up and down drag events as shown in FIG 6B, and right and left drag events as shown in FIG 6C, while the finger (or stylus, etc.) touches on the touch panel.

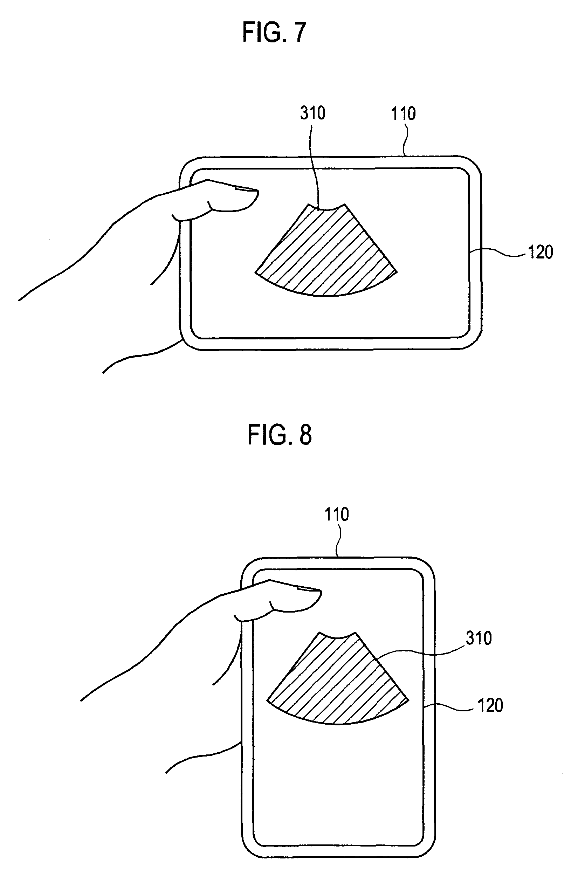

FIGS. 7 and 8 are schematic diagrams showing examples of displaying an ultrasound image according to the status of a hand-held ultrasound system 100 held by the user. FIG. 7 shows an example wherein the user holds the ultrasound system 100 in a longitudinal direction, while FIG. 8 shows an example in which the user holds the ultrasound system 100 in a vertical direction.

In one embodiment, the sensors installed on the respective edges of the body 110 may sense any touch caused by the user's hand to thereby output the detection signal indicative of which edge is held. The control unit 116, which is coupled to the rotation detection unit 115 to receive the detection signal, may be operable to control a display operation of the ultrasound image on the touch screen 120.

Also, the detecting unit 115 may detect whether the ultrasound system 100 is rotated within the first angle range from 0-45 degrees or the second angle range from 45-90 degrees by using the gravity sensor installed on the body 110. If the ultrasound system 100 is rotated within the first range, then the detecting unit 115 may be operable to output a first detection signal. Also, if the ultrasound system 100 is rotated within the second angle range, then the detecting unit 115 may be operable to output a second detection signal.

If the first detection signal is outputted from the rotation detection unit 115, then the control unit 116 may be operable to control an operation of the hand-held ultrasound system 100 such that the ultrasound image 310 formed by the ultrasound image forming unit 113 is displayed on the touch screen 120 as shown in FIG. 7. Also, if the second detection signal is outputted from the rotation detection unit 115, then the control unit 116 may be operable to control an operation of the hand-held ultrasound system 100 such that the ultrasound image 310 formed by the ultrasound image forming unit 113 is displayed on the touch screen 120 as shown in FIG. 8.

Although it is described above that the user holds the hand-held ultrasound system 100 by a left hand, the holding of the hand-held ultrasound system 100 may not be limited thereto. In one embodiment, the user may also hold the hand-held ultrasound system 100 by using a right hand. In such a case, the touch screen menu may be displayed on a right side of the touch screen 120.

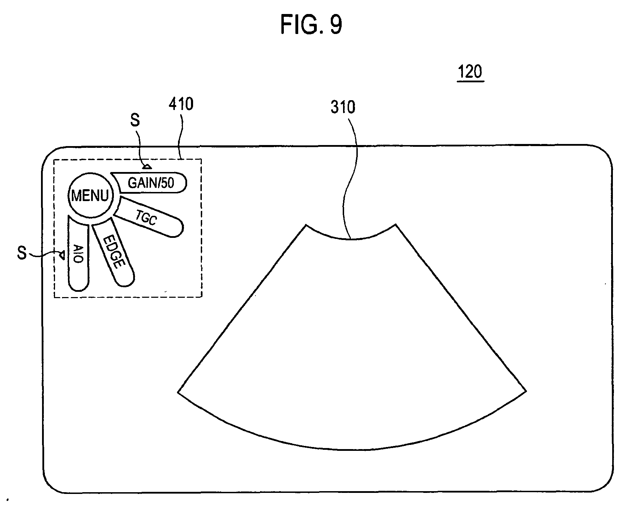

If the user initially touches an arbitrary location on the touch screen 120, then the control unit 116 may be operable to control the touch screen 120 such that the preset touch screen menu for graphic user interface is displayed on the touch screen 120, as shown in FIG. 9. In one embodiment, the control unit 116 may be operable to control the touch screen 120 such that the touch screen menu 410 is displayed at the location where the user touches. The touch screen menu 410 may be displayed on the second display region 122. The touch screen menu 410 may include a plurality of items, which may be represented by soft buttons, necessary for image optimization such as gain, time gain compensation (TGC), edge enhancement, auto image optimization (AIO), frame rate, etc. The displayed items may be changeable according to a user request. For example, the touch screen menu 410 may be set to include items such as gain, TGC, edge enhancement and AIO, as illustrated in FIG. 9. The touch screen menu 410 may further include a scroll S to display other items. The items included in the touch screen menu may be changed according to diagnostic modes or user input instructions.

If the user clicks the scroll S in the touch screen menu, the control unit 116 may be operable to control the touch screen 120 such that another item is displayed. Also, in one embodiment, the user may drag the scroll S to display another item. In such a case, the drag event may be performed in a circular direction.

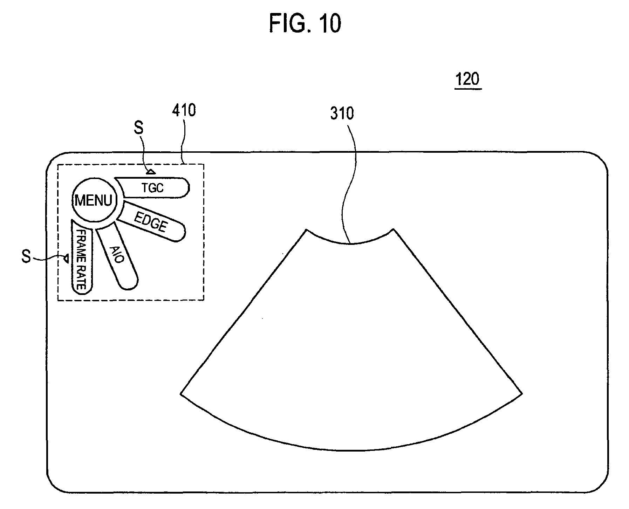

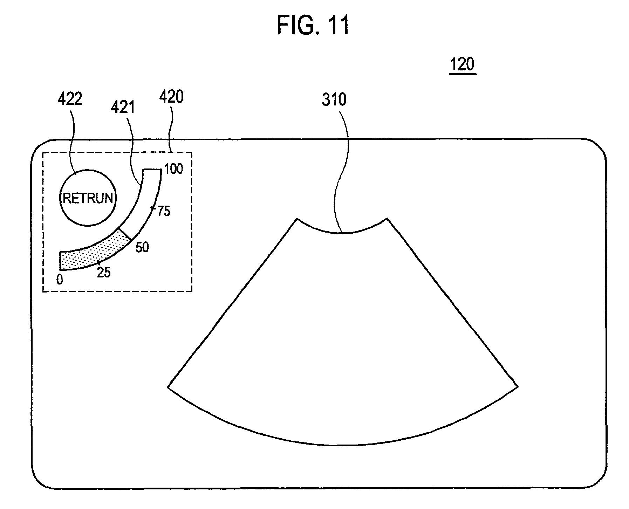

In one embodiment, the touch screen menu may include a main menu and a plurality of sub menus associated with the respective items included in the main menu. If a user taps one of the items in the main menu 410 for image optimization, i.e., clicks one item to select a desirable image optimization function, then the control unit 116 may be operable to control the touch screen 120 such that a preset sub menu 420 associated with the selected item in the main menu 410 may be displayed on the touch screen 120. The sub menu 420 may be provided with graphic user interface capable of adjusting an image parameter associated with the selected item. For example, if the user clicks gain in the main menu 410 shown in FIG 10, then the control unit 116 may be operable to control the touch screen 120 such that the preset sub menu 420 including a gain adjusting bar 421 for gain adjustment is displayed on the touch screen 120, as shown in FIG 11. The sub menu 420 may be also displayed on the second display region 122 of the touch screen 120. In one embodiment, the sub menu 420 may further include a return button to return to the main menu 410. Although it is described above that the sub menu 420 corresponding to the gain item includes a gain adjusting bar 421 for gain adjustment as an example, the setting of the sub menu 420 may not be limited thereto. The sub menu 420 may be set in various ways according to the user's tendency.

If the user adjusts an image parameter on the sub menu 420 through the click and/or drag events, then the control unit 116 may be operable to apply the adjusted image parameter to an ultrasound image for image optimization. Further, if the user selects the back soft button through the click events, then the touch screen 120 may display the previous graphic user interface, i.e., the main menu 410 under the control of the control unit 116.

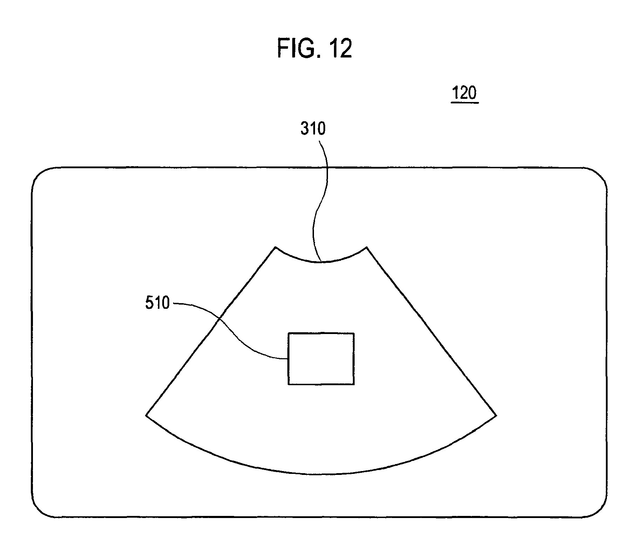

FIG 12 is a schematic diagram showing an example of setting a region of interest (ROI) on the ultrasound image displayed on the touch screen 120. If the user inputs a user instruction for setting ROI on an ultrasound image 310 displayed on the touch screen 120, then the control unit 116 may be operable to control the touch screen 120 such that a predetermined ROI 510 is set on the ultrasound image 310, as shown in FIG. 12. The input of the user instruction for setting the ROI may be achieved by touching a desirable location within the first display region 121. In one embodiment, the ROI may be set on the first display region 121 with reference to the location where the user touches. The border of the ROI 510 may be adjusted through the drag events in the up, down, right and left directions.

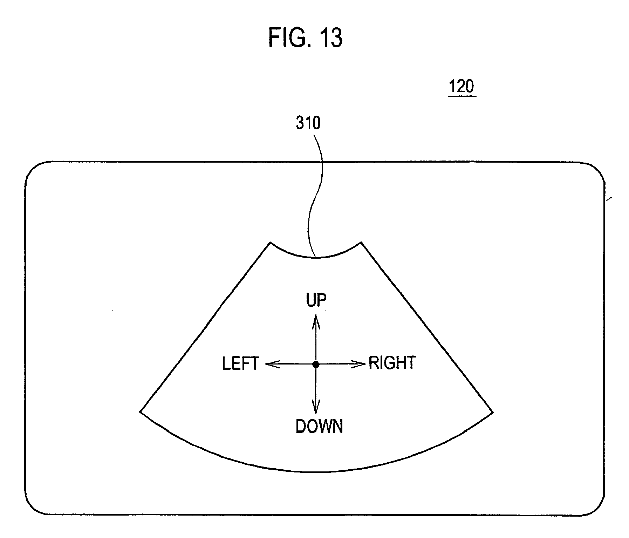

FIG 13 is a schematic diagram showing an example of selecting one of the ultrasound images and one of the ultrasound data through the touch and drag events. If the user inputs a user instruction for selecting one of the ultrasound images, then the control unit 116 may be operable to access the storage unit 114 to read out a corresponding ultrasound image. In one embodiment, the user instruction for selecting one of the ultrasound images may be achieved through touch and drag events in up and down directions on the first display region 121. If the user touches and drags in an up direction on the first display regions 121, then the control unit 116 may be operable to read out an ultrasound image, which is formed prior to a currently displayed ultrasound image. Also, if the user touches and drags in a down direction on the first display regions 121, then the control unit 116 may be operable to read out an ultrasound image, which is formed next to a currently displayed ultrasound image.

If the user inputs a user instruction for selecting one of the ultrasound data, then the control unit 116 may be operable to access the storage unit 114 to read out a corresponding ultrasound data. In one embodiment, the user instruction for selecting one of the ultrasound images may be achieved through the touch and drag events in the left and right directions on the first display region 121. If the user touches and drags in the left direction on the second display regions 121, then the control unit 116 may be operable to read out an ultrasound data, which is formed prior to an ultrasound data corresponding to the currently displayed ultrasound image. Also, if the user touches and drags in the down direction on the first display regions 121, then the control unit 116 may be operable to read out an ultrasound data, which is formed next to an ultrasound data corresponding to the currently displayed ultrasound image. In such a case, the read-out ultrasound data is processed by the ultrasound image forming unit 113 to form an ultrasound image. The formed ultrasound image may be displayed on the second display region 121 of the touch screen 120.

In the above embodiment, it has been described that the touch screen menu 410 includes the items for image optimization as the main menu. However, the touch screen menu 410 may not be limited thereto. The touch screen menu 410 may be set to include items for measuring a size of the target object, items for selecting diagnostic departments, items for selecting diagnostic modes and the like.

Also, it is described above that the ultrasound image is displayed only on the first display region 121. However, when the user instruction for displaying the ultrasound image on a full screen is inputted or any instruction is not inputted for a predetermined time duration, the control unit 116 may be operable to control the touch screen 120 such that the ultrasound image is displayed on a full screen including the first and second display regions. In such a case, if any user instruction is inputted, then the control unit 116 may be operable to control the touch screen 120 such that the ultrasound image is displayed only on the first display region 121 and the touch screen menu 410 is displayed on the second display region 122.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A hand-held ultrasound system (100), comprising:
an ultrasound probe configured to transmit and receive ultrasound signals to and from a target object to thereby output receive signals;
a body (110) sized to be adapted to be held by a user with only a single hand and having processing elements for forming ultrasound images based on the receive signals and provide a preset touch screen menu; and
a touch screen (120) mounted on the body including a display and a touch panel with a touch response surface, said touch screen (120) being configured to display the ultrasound images and the preset touch screen menu for allowing the user to input user instructions,
**characterized in that**
a display region of the touch screen (120) is partitioned into a first display region (121) disposed at a center portion of the display region to display the ultrasound image and a second display region (122) disposed at both sides of the first display region (121) to display the preset touch screen menu to be touched by a finger of a holding hand of the user, and
the user instructions is adapted to input via the touch screen menu displayed in the second display region (122) by circular drag event using the finger of the holding hand of the user.

2. The hand-held ultrasound system (100) of Claim 1, wherein the body (110) includes:
an ultrasound data forming unit (112) configured to form ultrasound data based on the receive signals;
an ultrasound image forming unit (113) configured to form ultrasound images based on the ultrasound data;
a storage unit (114) for storing the ultrasound data and the ultrasound images; and
a control unit (116) configured to control a display operation of the touch screen.

3. The hand-held ultrasound system (100) of Claim 1, wherein the preset touch screen menu includes a main menu including a plurality of items for image optimization and a sub menu for adjusting an image parameter corresponding to each of the items.

4. The hand-held ultrasound system (100) of Claim 1, wherein the body further includes a detecting unit operable to detect a status of the hand-held ultrasound system held by the user to thereby output a detection signal, and wherein the control unit is operable to control the display of the ultrasound image and the preset touch screen menu on the touch screen in response to the detection signal.

5. The hand-held ultrasound system (100) of Claim 4, wherein the detecting unit includes sensors mounted on respective edges of the body for detecting any touch caused by a user's hand to thereby output a detection signal indicative of which edge is held.

6. The hand-held ultrasound system (100) of Claim 5, wherein the control unit is configured to control the display of the ultrasound images and the present touch screen menu in response to the detection signal.

7. The hand-held ultrasound system (100) of Claim 4, wherein the detecting unit includes a gravity sensor for detecting the status of the hand-held ultrasound system held by the user to thereby output a detection signal.

8. The hand-held ultrasound system (100) of Claim 7, wherein the control unit is configured to control the display of the ultrasound images and the present touch screen menu in response to the detection signal.

## Patentansprüche

1. Tragbares Ultraschallsystem (100), welches folgendes aufweist:
Eine Ultraschallsonde, die dafür vorgesehen ist, Ultraschallsignale zu und von einem Zielobjekt zu übertragen bzw. zu empfangen, um dadurch Empfangssignale auszugeben;
einen Körper (110), der eine derartige Größe aufweist, dass es möglich ist, denselben durch einen Benutzer mit nur einer einzelnen Hand zu halten, und der Verarbeitungselemente zum Bilden von auf den Empfangssignalen basierenden Ultraschallbildern und zum Erzeugen eines voreingestellten Touchfeldmenüs aufweist;
einen Touchscreen (120), der auf dem Körper montiert ist und eine Anzeige und ein Bedienfeld mit einer auf Berührungen reagierende Oberfläche aufweist, wobei der Touchscreen (120) dafür vorgesehen ist, die Ultraschallbilder und das voreingestellte Touchscreenmenü anzuzeigen, um dem Benutzer zu ermöglichen, Benutzereingaben einzugeben,
**dadurch gekennzeichnet,**
**dass** ein Anzeigebereich des Touchscreen (120) in einen ersten Anzeigebereich (121), der an einem mittleren Abschnitt des Anzeigebereichs angeordnet ist, um das Ultraschallbild anzuzeigen, und einen zweiten Anzeigebereich (122) unterteilt ist, der an beiden Seiten des ersten Anzeigebereichs (121) angeordnet ist, um das voreingestellte Touchscreenmenü anzuzeigen und um von einem Finger einer gehaltenen Hand des Benutzers berührt zu werden, und
die Benutzereingaben dafür vorgesehen sind, über das Touchscreenmenü eingegeben zu werden, das in dem zweiten Anzeigebereich (122) angezeigt wird, und zwar durch einen kreisförmigen Ziehvorgang unter Verwendung des Fingers der gehaltenen Hand des Benutzers.

2. Tragbares Ultraschallsystem (100) nach Anspruch 1, wobei der Körper (110) folgendes aufweist:
Eine Ultraschalldatenerzeugungseinheit (112), die dafür vorgesehen ist, Ultraschalldaten basierend auf den Empfangssignalen zu erzeugen;
eine Ultraschallbilderzeugungseinheit (113), die dafür vorgesehen ist, Ultraschallbilder basierend auf den Ultraschalldaten zu erzeugen;
eine Speichereinheit (114) zum Speichern der Ultraschalldaten und der Ultraschallbilder; und
eine Steuereinheit (116), die dafür vorgesehen ist, eine Anzeigeeingabe des Touchscreen zu steuern.

3. Tragbares Ultraschallsystem (100) nach Anspruch 1, wobei das voreingestellte Touchscreenmenü ein Hauptmenü mit einer Vielzahl von Elementen zur Bildoptiemierung und ein Untermenü zum Einstellen eines Bildparameters, das jedem der Elemente entspricht, aufweist.

4. Tragbares Ultraschallsystem (100) nach Anspruch 1, wobei der Körper des weiteren eine Erkennungseinheit aufweist, die in der Lage ist, einen Status des von dem Benutzer gehaltenen, tragbaren Ultraschallsystems zu detektieren, um dadurch ein Erkennungssignal auszugeben, und wobei die Steuereinheit in der Lage ist, die Anzeige des Ultraschallbilds und das voreingestellte Touchscreenmenü auf dem Touchscreen als Reaktion auf das Erkennungssignal zu steuern.

5. Tragbares Ultraschallsystem (100) nach Anspruch 4, wobei die Erkennungseinheit Sensoren aufweist, die an jeweiligen Rändern des Körpers montiert sind, um jede von der Hand eines Benutzers bewirkte Berührung zu erkennen, um dadurch ein Erkennungssignal auszugeben, das anzeigt, welcher Rand gehalten wird.

6. Tragbares Ultraschallsystem (100) nach Anspruch 5, wobei die Steuereinheit dafür vorgesehen ist, die Anzeige der Ultraschallbilder und das voreingestellte Touchscreenmenü als Reaktion auf das Erkennungssignal zu steuern.

7. Tragbares Ultraschallsystem (100) nach Anspruch 4, wobei die Erkennungseinheit einen Gravitätssensor aufweist, um den Status des von dem Benutzer gehaltenen, tragbaren Ultraschallsystems zu erkennen, um dadurch ein Erkennungssignal auszugeben.

8. Tragbares Ultraschallsystem (100) nach Anspruch 7, wobei die Steuereinheit dafür vorgesehen ist, die Anzeige der Ultraschallbilder und des voreingestellten Touchscreenmenüs als Reaktion auf das Erkennungssignal zu steuern.

## Revendications

1. Système ultrasonique portable (100) comportant :
une sonde ultrasonique configurée pour transmettre et recevoir des signaux ultrasoniques vers et provenant d'un objet cible pour émettre ainsi des signaux reçus; et
un corps (110) dimensionné pour s'adapter à être tenu par un utilisateur d'une seule main et comportant des éléments opérationnels pour former des images ultrasoniques basées sur les signaux reçus et fournissant un menu prédéterminé d'écran tactile ; et
un écran tactile (120) monté sur le corps comportant un affichage et une panneau de touches avec une surface de touche réponse, ledit écran tactile (120) étant configuré pour afficher les images ultrasoniques et le menu prédéterminé d'écran tactile, pour permettre à l'utilisateur d'introduire des instructions d'utilisateur,
**caractérisé en ce que**
une zone d'affichage de l'écran tactile (120) est partitionnée en une première zone d'affichage (121) disposée dans une partie centrale de la zone d'affichage, pour afficher l'image ultrasonique et une seconde zone d'affichage (122) disposée sur les deux côtés de la première zone d'affichage (121), pour afficher le menu prédéterminé d'écran tactile pouvant être touché par un doigt de la main portante de l'utilisateur, et
les instructions de l'utilisateur sont adaptées pour être introduites via le menu de l'écran tactile affiché sur la seconde zone d'affichage (122) par une dérive circulaire faite avec le doigt de la main porteuse de l'utilisateur.

2. Système ultrasonique portable (100) selon la revendication 1 dans lequel le corps (110) comporte :
une unité (112) de constitution des données ultrasoniques configurée pour former des données ultrasoniques basées sur les signaux reçus ;
une unité de formation d'image (113) configurée pour former des images ultrasoniques basées sur les données ultrasoniques ;
une unité de stockage (114) pour mémoriser les données ultrasoniques et les images ultrasoniques ; et
une unité de commande (116) configurée pour contrôler une opération d'affichage sur l'écran tactile.

3. Système ultrasonique portable (100) selon la revendication 1, dans lequel l'écran tactile comprend un menu principal comportant une pluralité d'entrées pour optimiser les images et un sous-menu pour ajuster les paramètres d'image correspondant à chaque entrée.

4. Système ultrasonique portable (100) selon la revendication 1, dans lequel le corps comporte en outre une unité de détection agencée pour détecter un état du système ultrasonique portable tenu par un utilisateur afin de produire un signal de détection émis, et dans lequel l'unité de commande peut être commandée pour contrôler l'affichage de l'image ultrasonique et le menu prédéterminé d'écran tactile sur l'écran tactile en réponse au signal de détection.

5. Système ultrasonique portable (100) selon la revendication 4, dans lequel l'unité de détection comprend des capteurs montés sur des bords respectifs du corps pour détecter l'appui de la main d'un utilisateur et émettre ainsi un signal de détection indiquant quel bord est tenu.

6. Système ultrasonique portable (100) selon la revendication 5, dans lequel l'unité de commande est configurée pour commander l'affichage des images ultrasoniques et le menu prédéterminé d'écran tactile en réponse au signal de détection.

7. Système ultrasonique portable (100) selon la revendication 4, dans lequel l'unité de détection comporte un capteur de gravité pour détecter l'état du système ultrasonique portable tenu par l'utilisateur afin d'émettre ainsi un signal de détection.

8. Système ultrasonique portable (100) selon la revendication 7, dans lequel l'unité de commande est configurée pour commander l'affichage des images ultrasoniques et le menu prédéterminé d'écran tactile en réponse à un signal de détection.
